# EUROPEAN PATENT SPECIFICATION

(11) **EP 2 498 092 B1**
(45) Date of publication and mention of the grant of the patent: **22.07.2015**
(21) Application number: 12158910.5
(22) Date of filing: 09.03.2012
(51) Int. Cl.: G01N 33/497, G01N 21/65

(54) **A vehicle interlocking method based on detection of analytes in exhaled breath**
Fahrzeugverriegelungsverfahren basierend auf dem Nachweis von Drogen in der Ausatemluft
Procédé d'interverrouillage de véhicule basé sur la détection de drogues dans le souffle expiré

(30) Priority: 09.03.2011 US 201161451001 P; 09.03.2011 EP 11157565
(43) Date of publication of application: 12.09.2012
(73) Proprietor: Sensa Bues AB, 141 57 Huddinge (SE)
(72) Inventor: Beck, Olof, 132 44 Saltsjöö-Boo (SE); Hammarlund, Bo, 191 40 Sollentuna (SE)
(74) Representative: KIPA AB

(56) References cited:
- WO-A1-03/057521
- WO-A1-2011/029888
- MARKS P: "Taking on the drugged and drunk drivers", NEW SCIENTIST, REED BUSINESS INFORMATION, SURREY, GB, vol. 188, no. 2528, 3 December 2005 (2005-12-03), pages 28-29, XP008137810, ISSN: 0262-4079

## Description

### Field of the Invention

This invention pertains in general to the field of interlocking vehicles for preventing drivers under drug influence to operate the vehicle. The method is based on collecting a sample from exhaled breath of a subject, and for detecting the presence or determining the quantitative amount of analytes in the breath sample. The analytes are for instance drug substances in the exhaled breath. More particularly, the invention relates to using Surface Enhanced Raman spectroscopy in a sensor for detecting the analytes from the exhaled breath.

### Background of the Invention

It is known that exhaled breath is commonly used in alcohol testing and today's technology makes it possible to perform on-site breath testing with legally defensible results using electrochemical sensory. An emerging technology is using infrared spectroscopy.

However, testing for other illicit drugs of abuse still requires blood or urine samples. Alternatively specimens comprising hair, sweat or oral fluid could be used. Blood sampling is invasive and requires medically trained personnel, why test subject often have to be transported to a hospital for sampling. This is time and effort consuming. With long lead times the test result will be too old. Urine sampling is considered intruding on personal integrity and must be done under supervision of a nurse or a doctor. Even other issues related to samples and specimen taken from a subject to be tested arise. For instance for blood samples, and especially for urine samples are at risk of the subject exchanging the samples or using clean samples from another subject to avoid being discovered with traces of illicit drugs.

Result from a study related to this topic and performed on Ireland can be found in: Results and conclusions from Injury Prevention 2006;12:404-408. doi: 10.1136/ip.2006.013177. 33.1 % of the drivers under the legal limit for alcohol tested positive for one or more of the relevant drugs, and the corresponding figures of drivers over the limit was 14.2. Among drivers who had minimal blood alcohol levels, 67.9% were taking at least one type of drug. The prevalence of taking drugs reduced steadily as alcohol concentrations increased, but still remained as high as 11.1 % for drivers with blood alcohol concentrations .200 mg/100 ml. Being under the limit for alcohol, stopped in a city area, stopped between 6 am and 4 pm, or 4 pm and 9 pm, and being of a younger age were each independently associated with drug positivity.

Conclusions of the study point out the serious need for a readily available drug test in addition to today's alcohol tests. There are immediate implications for the evidential breath alcohol program and for checkpoints; in the event of a nil or low alcohol reading being obtained, a separate blood or urine specimen should be sought for analysis, which is currently non-routine. However, obtaining blood or urine specimen as a routine test for all drivers in regular traffic controls is not a feasible alternative due to the issues pointed out above.

Another investigation related to this topic is described in: Investigate of the prevalence and characteristics of abusive drug exposure among non-fatal motor vehicle driver casualties in Hong Kong. (Hong Kong Med J 2010; 16:246-51). The Setting for this study was a Designated trauma centre/regional accident and emergency department in Hong Kong. Investigated subjects were Non-fatal motor vehicle driver casualties who presented to the trauma centre from 1 January 2007 to 31 December 2007.

Results from drug screening that was performed in 395 injured drivers show 10% of whom tested positive for the drugs of interest. Ketamine was the most commonly detected abusive substance (found in 45% of the subjects). A significantly higher proportion of young drivers (aged <25 years) screened positive (odds ratio=2.3; 95% confidence interval, 1.0-5.2; P=0.04), with the rate being 21%.

The presence of these drugs in urine was related to the time of occurrence of the crash; those occurring between midnight and dawn revealed a trend towards a higher proportion of casualties testing drug-positive (odds ratio=2.2; 95% confidence interval, 0.9-5.3; P=0.07). There were no significant differences in the frequency of persons testing positive for the screened drugs with respect to gender, class of motor vehicle driven, or the day of the week on which the crash occurred

This study further supports the urgent need for a convenient, reliable and quick detection of drugs in subjects. An apparatus, system and/or method would be advantageous which allows at least for a pre-screening of subjects to identify subjects under the influence of drugs. These subjects may then further investigated, e.g. by obtaining blood or urine specimens for analysis.

WO 03/057521 discloses a breathanalyzer connected to a vehicle interlock system.

Intermediate document WO 2011/029888 discloses the detection of a drug substance in exhaled breath, using a sensor having a SERS-active layer.

In addition, there is a need for being able to detect other molecules from exhaled breath as well. For instance biomarker compounds indicative of various kinds of diseases would be desirable to being able to detect.

However, as there are a multitude of different analytes in exhaled breath, most in very low amounts or only as traces, it is a challenge to have a measurement system or method that is sufficiently sensitive to discern between all these different analytes.

Thus, there is a need to provide a non-invasive, not-specimen based method for detecting the presence or determining the quantitative amount of analytes, in particular at least one drug substance in a subject.

Hence, an improved method for on-site sampling of a subject for analytes, in particular drug substances is desired. Such a method for sampling the subject for illicit drugs of abuse and/or medical drugs would be desired. The method should be efficient, non-bulky, user friendly both for operators and the subject. It should further be not intruding and not invasive. It should preferably be able to discern between various analytes.

### Summary of the Invention

In accordance with the present invention, the use of an SERS-active substrate or layeris provided as a drug interlock for vehicles based on exhaled breath directed onto said SERS-active substrate. A detected drug substance in exhaled breath of a subject provides a signal for a control unit of said vehicle to lock said vehicle from use. Locking may be provided for at least a pre-determined time after drug detection. Alternatively, or in addition, repeated exhaled breath test where no drugs are detected may also unlock the vehicle. The drug interlock is a combined drug and alcohol interlock.

Further embodiments of the invention are defined in the dependent claims.

Embodiments provide for in-situ measurements providing measurement results directly from exhaled breath.

Identification of molecules is thus detectable based on exhaled breath samples. Some embodiments allow for quantitative determination of the chemical compounds found in exhaled breath.

Some embodiments provide for sufficient sensitivity to discern between different analytes in exhaled breath samples. Measurements systems and methods that are selectively providing Raman spectra for pre-defined chemical compounds or analytes in exhaled breath are provided.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

Fig. 1 is a schematic illustration that shows an embodiment of the SERS-sensor with a first SERS-active layer and an optional analyte permeable or analyte selectively permeable layer;
Fig. 2 is a schematic illustration that shows another embodiment of the SERS-sensor similar to the previous but with a SERS-active layer being a slurry or a mixture of at least one SERS-active material and at least on analyte permeable material;
Fig. 3 is a schematic illustration that shows an alternative arrangement of the SERS-sensor;
Fig. 4a is a graph that shows three Raman spectra;
Fig. 4b is a graph that shows two SERS spectra of spiked exhaled breath, whereof one is spiked with amphetamine;
Fig. 5 is a schematic illustration illustrating an embodiment of a system based on a SERS-sensor;
Fig, 6 is a flow-chart illustrating a first method for using a SERS-sensor;
Fig. 7 is a flow-chart illustrating a second method for using a SERS-sensor system;
Fig. 8 is a flow-chart illustrating the code segments of a computer-program;
Fig. 9a and b are schematic illustrations of an SiO2 planar multimode waveguide with a slot. The SERS-layer comprising SERS-particles are located in the slot;
Fig. 10 shows a schematic illustration of a plate of porous material.;
Fig. 11 is a flowchart illustrating an example for a method of producing a slot for a SERS-sensor; and
Fig. 12 is a flowchart illustrating another example for a method of producing a slot for a SERS-sensor.

### Description of embodiments

A vehicle interlock method is disclosed for preventing a subject under drug influence to operate a vehicle, said unit including at least one sensor, and a unit for analyzing if at least one analyte is present in exhaled breath of a subject based on measurement of said exhaled breath by means of said sensor, wherein said sensor is a SERS-sensor for providing a SERS-emitted signal from said analyte; a unit for analyzing said analyte is present in said exhaled breath of said subject based on said SERS-emitted signal of said SERS-sensor; and a unit for interlocking said vehicle if said analyte is detected in said exhaled breath.

The SERS-sensor has a light source, a light detector, at least one optical filter, and at least one planar multimode waveguide having a slot, and a collecting surface having at least one Surface Enhanced Raman Spectroscopy (SERS)-active cladding layer being refractive index matched with said planar waveguide, wherein said collecting surface is arranged as an outer surface in said slot for contact with said exhaled breath, such that at least traces of said analyte in said exhaled breath can come in close contact said SERS-active layer, and wherein said at least one planar waveguide is coupled to said light source and said detector for providing an optic field from said light source and an evanescent mode to interact when said analyte is in contact with said SERS-layer.

The waveguide and said slot are arranged to enhance both said optic field and said evanescent mode interaction and detection of a SERS-emitted signal from said analytes by total internal reflection for detecting a Raman shift spectrum in the presence of said analyte and/or determining a quantitative amount of said analyte, such as at least one drug substance, from said exhaled breath.

The system has a gas conduit arranged to convey said exhaled breath to a SERS-active layer of said SERS-sensor. The waveguide may be made of SiO₂.

The collecting surface is preferably arranged for collecting a breath condensate or aerosol with said analyte.

The SERS-sensor has a SERS-active layer, and a second layer covering said SERS-active layer wherein said second layer is enhanced permeable for said analyte, such as by said second layer being made of an analyte permeable material selected from the group comprising silicone polymers and silica and silicone elastomers.

At least one SERS-active layer of said SERS-sensor may be provided as a mixture comprising at least one SERS-active material and at least one material selected from a polymer material group, such as including silicone elastomers.

The system may include a vehicle having a coupé and at least a seat and the subject is a driver or passenger in said coupé.

The system may comprise a mouthpiece for said subject providing said exhaled breath to said SERS-sensor, wherein said mouthpiece preferably is disposable and/or comprises bacterial killing substances, such as silver or copper particles.

The system may comprise a plurality of said SERS-sensors for arranging at different locations in said vehicle, wherein each of said SERS-sensors includes a light source and detector, or wherein said system comprises a light source and detector shared by a plurality of SERS-sensors, wherein said light source is coupled to optical transmitting means for transmitting light to an a SERS-active layer and the emitted SERS signal from said layer to said detector.

The optical means may comprise optical fibres arranged in an infrastructure for cables already present in the vehicle.

The SERS-sensor may comprise at least one Klarite substrate, wherein said Klarite substrates preferably is arranged to be replaced after having indicated the presence of an analyte for a specific number of times or when a predetermined time of use has expired.

The system may include a magazine with unused waveguides having at least one SERS-layer for said SERS-sensor, wherein said magazine is arranged to replace a used waveguide after the presence of an analyte has been detected by said used waveguide a specific number of times or when a predetermined time of use of said used waveguide has expired.

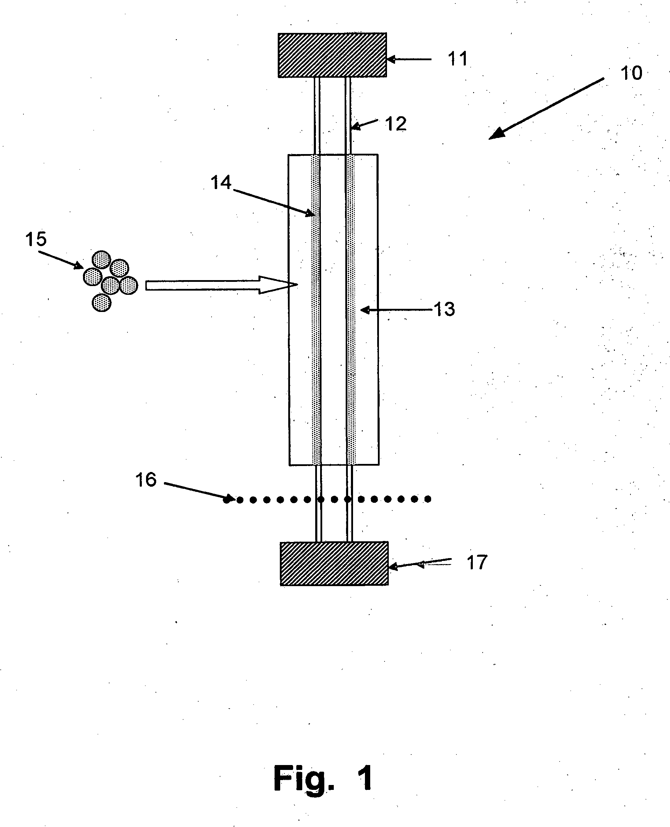

In Fig. 1 a part of a SERS-sensor 10 is shown. The sensor 10 is based on obtaining at least one Surface Enhanced Raman Spectrum (SERS) from exhaled breath. SERS is a surface technology, described in more detail below, enhancing Raman detection technology.

Thus embodiments of the SERS sensor allow for detection of one o more substances, even when present in very low amounts or concentrations.

Returning to Fig. 1, the sensor 10 is provided in form of a waveguide 12 having at least one SERS-active layer 14. The waveguide 12 may be an optical fiber.

The SERS-active layer 14 is arranged outside of the waveguide at the circumference of the waveguide 12 and is extending longitudinally along a portion of the outer surface thereof.

Each SERS-layer 14 comprises at least one SERS-active material. For each SERS-layer a different SERS-active material may be provided.

The SERS-active material should be of a metal material. The metal is preferably comprised in the group of gold or silver or copper or Platinum or palladium or any mixtures thereof. The SERS-active layer 14 could either be made of colloids or being a substrate similar to Klarite® The SERS-active material is provided as nanoparticles or nanoparticle aggregates, thus providing the SERS-active layer 14 with a nanoscale roughness.

To achieve a measurable effect by a SERS-based measurement system, as described below, a drug substance 15 found in exhaled breath needs to come into contact with the metal with a nanoscale roughness on the SERS-layer 14 surface, or with similar arrangements of the SERS active nanoparticles. The substance then determines the Raman spectrum registered by a light detector 17, which spectrum is emitted from the SERS-layer upon excitation from light of the light source 11.

The shape and size of the metal nanoparticles or nanoparticles aggregates or the roughness of the surface strongly affects the strength of the enhancement because these factors influence the ratio of absorption and scattering events.

The SERS-active layer 14 could optionally be at least over a surface portion thereof be covered, coated or encapsulated by an outer, encapsulating layer 13. The outer layer 13 may be made of either silicone polymers and/or silica and/or silicone elastomers or similar. The material that the encapsulating layer 13 is made of is chosen to be permeable for certain pre-defined analytes 15. The encapsulating layer 13 could therefore act as a barrier for unwanted analytes that shows a lower diffusion through the layer, thus work as a pre-selecting filter to select only the drug substances 15 of interest. Hence a clearer spectrum without unwanted peaks that could be an obstruction could be obtained. The coating 13 may reduce noise in the SERS-measurement.

Thus a SERS-sensor is configured for measurement of specific pre-defined drugs in exhaled breath, whereby the measurement is provided with an advantageous signal to noise ratio.

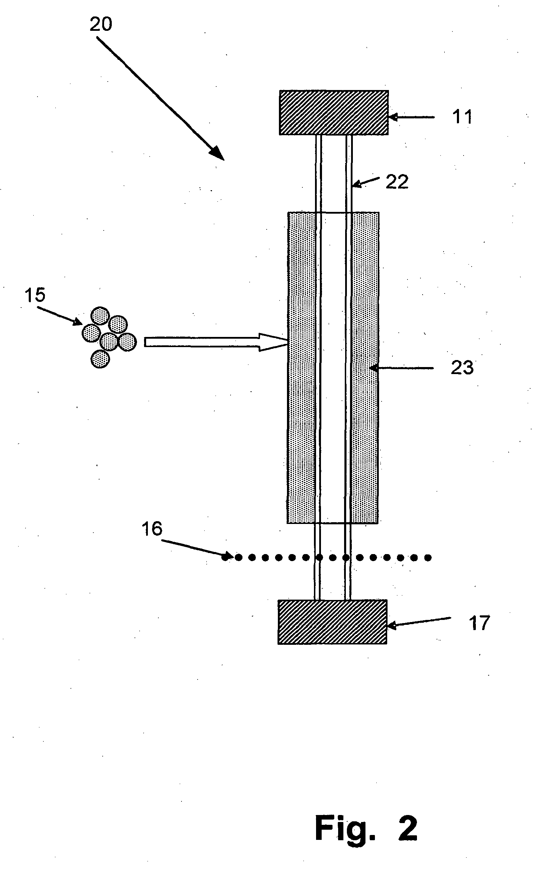

An alternative SERS-sensor 20 is illustrated in Fig. 2. The SERS-sensor has been produced by another method for applying the SERS-material onto the waveguide-surface 22, which result can be seen in Fig. 2. Here a slurry or a mixture of the SERS-material and a selectively permeable material are provided instead of a separate SERS-active layer and a pre-selecting filter material coating. The slurry or mixture may e.g. comprise the SERS-active nanoparticles and silicone polymers and/or silica and/or silicone elastomers and are provided for use as a single layer 23 on the waveguide 22 circumference.

The waveguide 22, preferably is a fiber, as mentioned above. More preferably the fiber is a mono-mode fiber.

The waveguide 22, 12 is arranged to have an inlet connected to a light source 11. The light source 11 could here be selected from a list comprising: lasers, laser diodes, light emitting diodes, hollow cathode lamps, Xenon arc lamps, deuterium lamps, metal halide, and plasma lamps.

When a fiber is used, it could have a cladding or the SERS-active layer 14, 23 could be used as a cladding. A criterion when providing the fiber 12, 22 with a cladding layer is that light can be transmitted or coupled from the core of the fiber 12, 22 to the surrounding SERS-active layer 14, 23. The light thus diverted out of the fiber core is then provided to excite the SERS-active layer 14, 23 of the outer cladding. The emitted light from the SERS-active layer 14, 23 is then transmitted or coupled back into the same fiber 12, 22 and spectrally analyzable to provide the drug measurement result. For a Multi-mode or graded-index fiber 12, 22 this can be done my allowing a part of the intensity of the reflected light between the core and the cladding to transmit to the SERS-active layer 14, 23. For a mono-mode fiber 12, 22 the evanescent field could be transmitted and used to excite to the SERS-active layer 14, 23. This could for example be done by modifying the cladding either by matching the refractive index of the cladding closer to the core or by making it thinner. For some embodiments it would even be an advantage to use the SERS-active layer 14, 23 as a cladding.

The light source 11 emits preferably light in the wavelength range between 0.3 and 1.5 µm but preferably in the range 0,5-1.2 µm, and even more preferably in the range 0.75-1.1 µm.

At the opposite end of the fiber 12, 22, from the inlet, is an outlet arranged in connection to an optical filter 16 and the light is then further directed onto a detector 17.

The filter 16 is used to remove the wavelength of the light source 11. The filter 16 may in addition or alternatively be used to limit the wavelength range to include only a wavelength range with peaks known to be strong for a specific drug substance or drug substances. By putting different filters 16 between the fiber 12, 22 and the detector 17 in a sequence after each other, different parts of the wavelength range can be obtained and recorded. Wherein each wavelength range, obtained using the optical filters 16, comprises information in the form of peaks, for at least one drug substance 15. In this manner, background noise is reduced and the measurement made robust and reliable.

The detector 17 may be selected from the list comprising: photocells, photodiodes, phototransistors, CCD, CMOS, photoelectric tubes and photomultipliers.

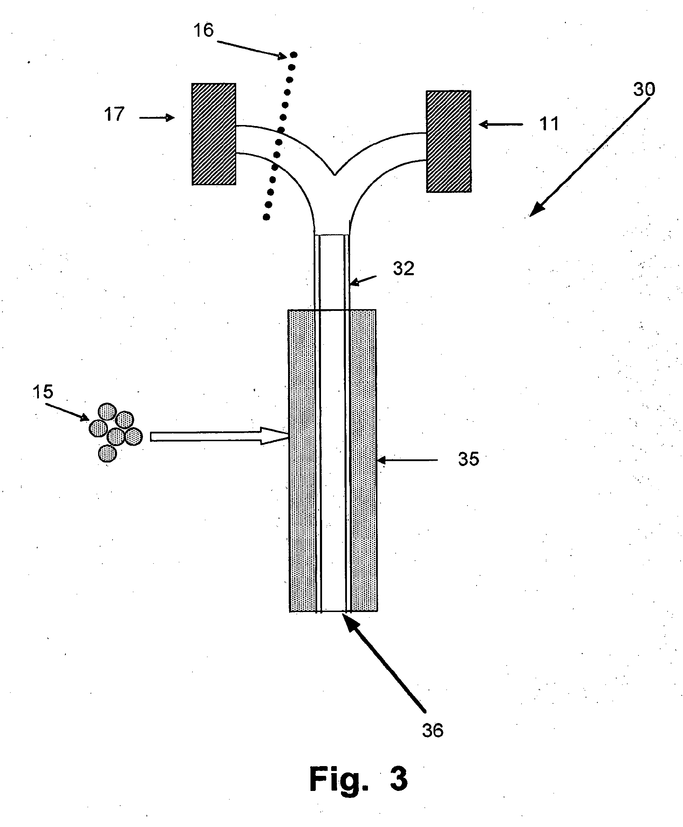

A further embodiment is illustrated in Fig.3 in form of a backscattering arrangement. A SERS sensor 30 is provided having one or more SERS-active layers 35. The SERS-layers 35, and if desired the drug substance permeable layers, are applied to the waveguide 32 and provided as described above. Here the detector 17, the at least one optical filter 16 and the light source 11 are located at the same side of the fiber 32 where light is both coupled in and out of the fiber 32. Thus one end of the waveguide 32 is both the excitation light inlet and the SERS layer reflected light outlet. At the distal end of the waveguide a mirror 36 or other highly reflective material can be placed to reflect the light back into the fiber 32. This will enhance the signal further since the SERS-layer 35 will be exposed to the excitation light twice.

The at least one waveguide is at least one optical fiber. The optical fiber is for instance made of silica glass or a suitable polymer. The fiber may be bent. In addition, the fiber may be coiled. Such embodiments provide for compact sensor arrangements with large available collecting surfaces.

The sensor device may comprise a plurality of fibers.

In some embodiments the collecting surface is arranged for collecting a breath condensate or aerosol with said drug. The sensor device may comprise a temperature control element that is arranged to keep said collecting surface at a temperature lower than 37 deg C to provide condensation of vapor in said exhaled breath. Other surface parts of the sensor device may be heated to a temperature higher than 37 deg C to avoid condensation of vapor in said exhaled breath. This allows for a controlled condensation at the collecting surface.

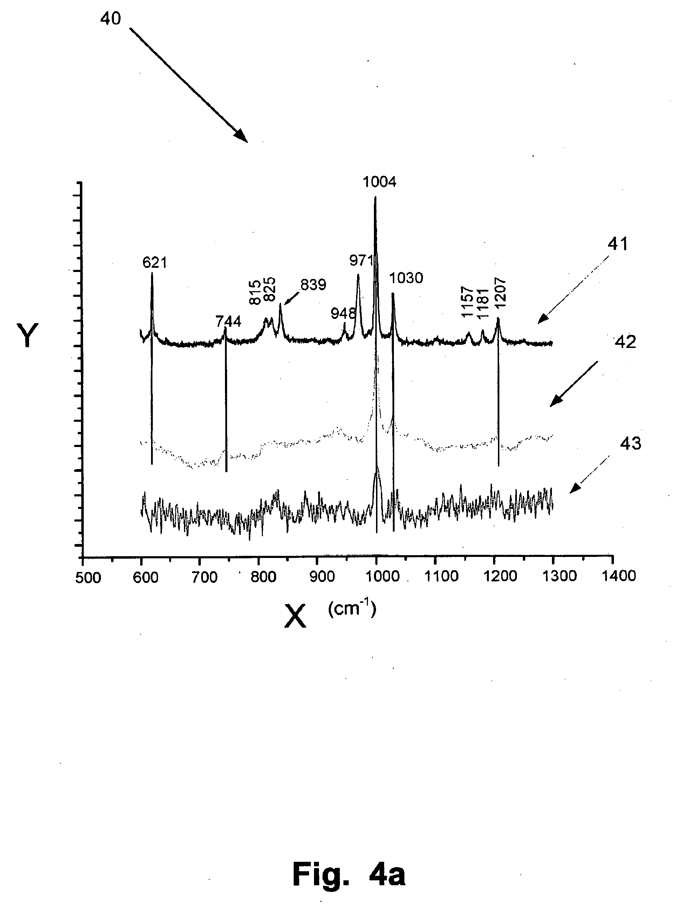

In Fig. 4a, a spectrum from a test on Klarite® is shown, see the curve 42. Klarite® substrates are commercially available from Renishaw Diagnostics, see http://www.renishawdiagnostics.com/en/12409.aspx. In more detail, the graph 40 illustrates a SERS spectrum obtained from detection of amphetamine by Raman spectroscopy utilizing irradiation light 785 nm. Normal (not enhanced) Raman spectrum of pure amphetamine powder 41 shows many peaks that can be used for identification, for example those positioned at 621, 1004, 1030 and 1207 cm⁻¹. SERS spectra are also obtained for diluted amphetamine solutions; one 42 corresponds to amphetamine adsorbed on gold nanostructured Klarite surface and one 43 corresponds to 1 µM amphetamine solution containing SERS active gold nanoparticles. A droplet of 2 µL amphetamine solution of 1 M was added to Klarite surface and after solvent evaporation a laser beam was focused on a micrometer sized spot to generate a characteristic spectrum 42. The amount of the drug that gives rise to spectrum 42 is estimated to be 0.3 pg.

The SERS spectra 40 of the drug substances of Fig. 4a shows a series of unique, high intensity peaks which give a fingerprint of the molecular structure of the tested drug substance. This feature of SERS allows the identification of unknown compounds in addition to their detection at low concentrations. Furthermore, the band positions and relative intensities of the SERS-spectra match closely to the bulk Raman signal.

Surface enhanced Raman spectroscopy (SERS), is used to identify amphetamine in exhaled breath. Using the SERS surface Klarite®, measurements of very low amounts, down to a few pg, of amphetamine have successfully shown that it is possible to measure the low concentration of amphetamine expected to be found in exhaled breath. Detection of amphetamine in exhaled breath is difficult because of interfering background signals from bio molecules in breath samples and impurities in solvents. The data indicates that it is possible to measure amphetamine in exhaled air, using SERS, from drug users if it is possible to reduce the background noise.

A multivariate data analysis was used, where the first derivates of unprocessed spectra were used. Ten breath samples without amphetamine, four breath samples spiked with 20-25 pg and 2 breath samples from drug users where used to build the model. The same samples and four more samples spiked with 100 pg amphetamine were used to calculate a Partial least square (PLS) model. Results indicated clearly that multivariate data analysis is suitable for separating persons under the influence of drugs from persons not having consumed drugs.

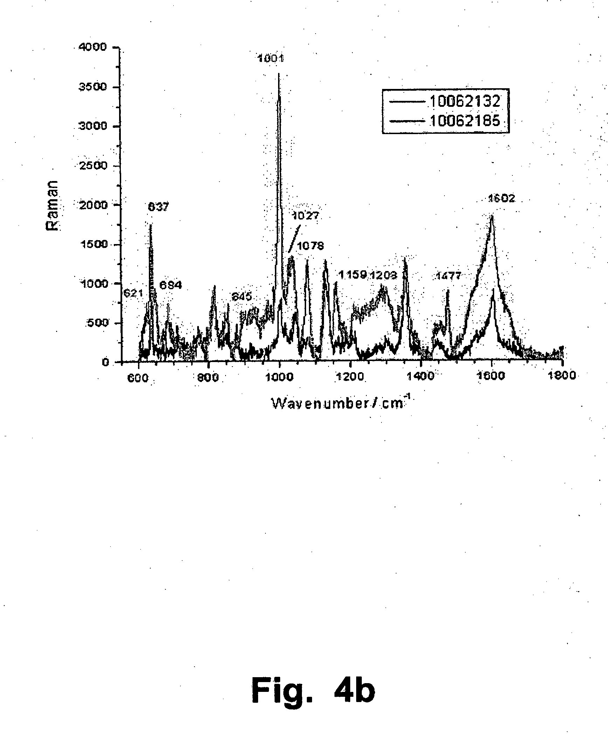

In Fig. 4b, some further SERS-spectra from a test on Klarite^{®} are shown. The spectra shows two spectrum one of exhaled breath without amphetamine and one spiked with 20 pg. The spiked spectra has a clearly visible peaks related to amphetamine.

In figure 4b SERS spectra are shown of exhaled air adsorbed on a filter. Both filters where eluted with organic solvents and one of the samples where spiked with 20 pg amphetamine (red spectrum). In conjunction with SERS measurement the samples where dissolved in pure deionized water and a droplet of 2 µL was added to a naked SERS active surface (Klarite). Before laser illumination of 785 nm (3 mW) the liquid droplet was evaporated and exhaled substances were adsorbed on the SERS active surface in dry condition. The spectra were performed on a HORIBA LabRam HR800 Raman spectrometer. It is obvious the Raman signatures are of complex nature where many peaks originating from different species are overlapping. However, figure 4b is indicative that in principle one could distinguish between drug containing and not containing exhaled air. One way is to build up a multivariate data model where a relevant training set is crucial. Most important spectral region in such pattern recognition working model depends on the target molecules but mostly it is lying in range of 600 - 1800 cm⁻¹. Sometimes high frequency region is of importance around 2300 and 3000 cm⁻¹. Figure 4b also says that it if the selectivity of the SERS surface is increased towards certain drug it should be much easier to judge any presence of target molecules. For example this could be achieved by mixing a polymeric layer with SERS active surfaces (or nanoparticles) possessing suitable physicochemical properties for diffusion certain class of analytes of interest.

Fig. 4 supports clearly the selectivity of SERS active surfaces for detecting certain chemical compounds from exhaled breath. This applies even though only very small amounts or traces of the specific compound are present in the exhaled breath. In addition, it has been shown that detection of traces of certain specific chemical compounds in exhaled breath is provided although many other chemical compounds are present in the exhaled breath and it would have been expected that measurement results were influenced. However, it was shown that measurement results for the desired specific compound under investigation are reliable and not disturbed by other compounds present in the in exhaled breath. Multiple SERS surfaces that are prepared to be selective for different chemical compounds may be combined to provide a multi parameter system in a single compact system.

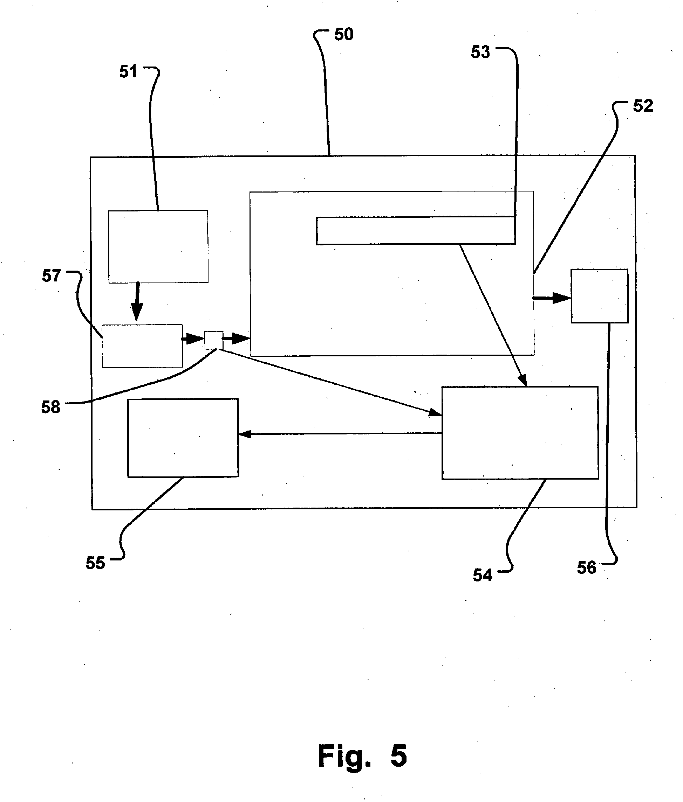

Fig. 5 is a schematic chart illustrating a vehicle 50 based on the invented sensor 53. A subject to be tested exhales into a mouth-piece or a mask 51, that is in communication with a collection chamber 52 via at least one inlet 57. The bold arrows in Fig. 5 illustrate a flow of the exhaled breath.

Optionally the inlet could be provided with a flow sensor 58 be arranged for measuring the flow of the exhaled breath and send the information to the control unit 54. Thus the flow over time and thus the volume of exhaled breath may be determined based on an output signal of the flow sensor 58. Hence, concentrations of substances in the exhaled breath may be determined.

In the collection chamber is at least one sensor 53 arranged for recording at least on SERS-spectrum. In use, the exhaled breath exits the chamber through the collection chamber's 52 at least one outlet 56. The at least one recorded spectrum is sent to the control unit 54 to be analyzed. The obtained result is prompted to the operator to subject via a user interface 55.

The user-interface 55 could also be used to tell the subject when the system is ready to be used or if any error has appeared, e.g. during the measurements or during the system's initialization phase.

Fig, 6 is a flow-chart illustrating the method 60 for using the SERS-sensor 61. First, the SERS-sensor 61 is provided. In the next step an exhaled breath sample is collected from a subject 62. This is done by exhaling breath onto the sensor 61. This maybe done directly or via a mouthpiece or a breathing mask. The next step is to make sure that the drug substances appears close enough 63 to the SERS-active layer so that the enhancement of the Raman spectra can occur.

It is also preferable if the exhaled breath can be in a high concentration since that will increase the concentrations of drug substance particles close to the at least one SER-active layer. This can be done by arranging the sensor 61 in a collecting chamber. When the at least one drug substance to be determined is in close proximity 63 to a SERS-active layer at least one spectrum can be recorded 64 using the sensor 61. The at least one spectrum is in use analyzed and during this analysis it can be determined if a drug substance is present and/or what drug substance is present and/or the concentration (quantitative amount) of the drug substance is determined.

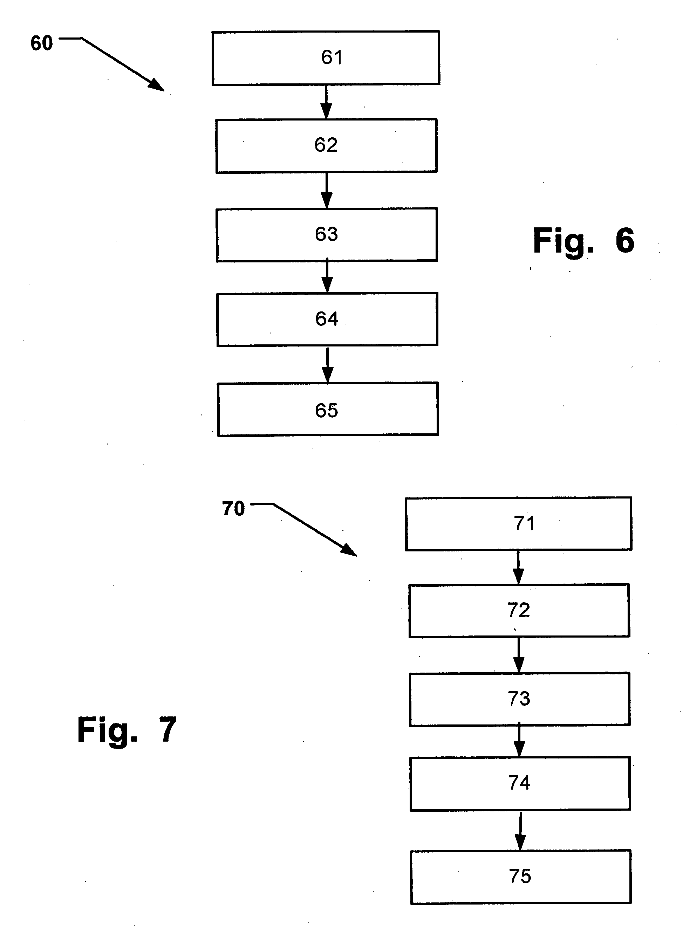

Fig. 7 shows a flow-chart illustrating a method 70 for detecting at least one drug substances in exhaled breath using a system comprising the aforementioned SERS-sensor.

The first step is an optional step of recording at least on background spectrum using the SERS-sensor 71.

The next step is to collect an exhaled breath sample in a collection chamber 72. The collection chamber can have any shape or size but is preferable a small tube with a small volume having the sensor arranged inside the tube. Hence there will be a high flow of exhaled breath through the tube leading to a concentration of drug particles around the sensor.

The next step is recording at least one SERS-spectrum 73 during a certain period of time. This period is determined so that a high signal-to-noise is achieved related to the time it takes for a subject to empty his/her lungs. The recorded spectrum is analyzed 74 using a software that is run on or part of a control unit 54. The Control unit is then prompting the subject with the results 75 via a user-interface. The user-interface could here be a red and a green lamp, a small display or the built in TFT screen of a car or a computer screen.

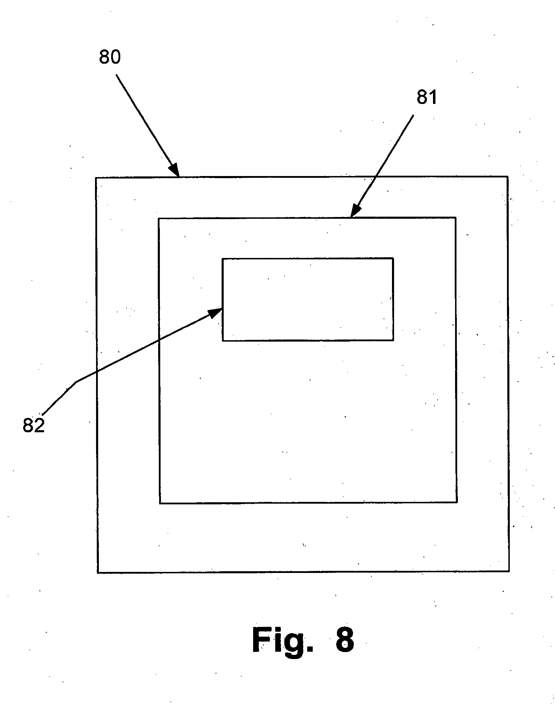

Fig. 8 is a flow-chart illustrating the code segments of the computer-program. 80 is a computer-readable medium with a computer program 81 having a plurality of code segments for analyzing spectral-data 82 and determining if a drug substance is presence in the exhaled breath.

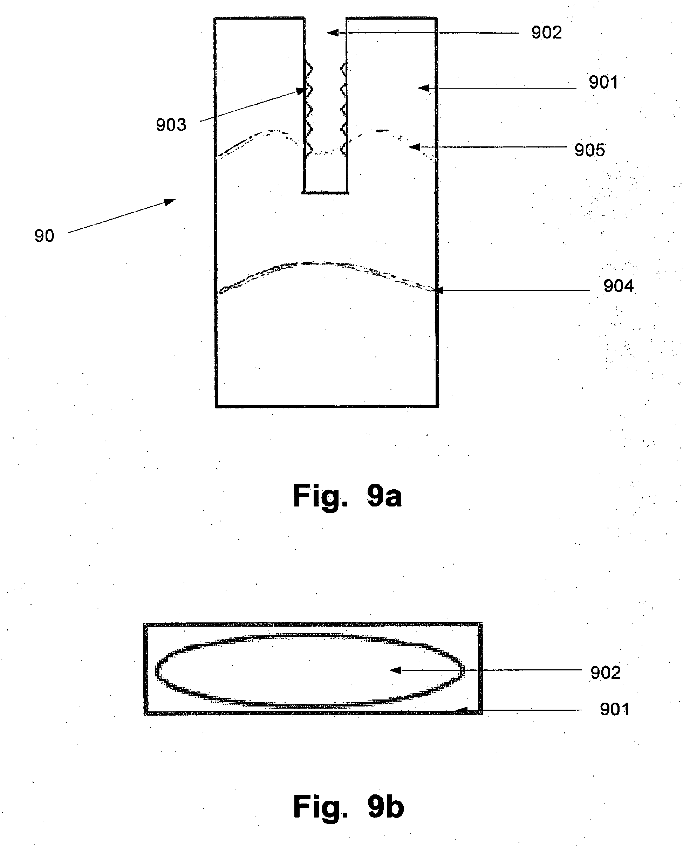

Fig. 9a (top view) and b (side view) illustrates a SERS-probe 90 based on a continuing and/or planar waveguide 901, preferably made of SiO2. The waveguide 901 has a slot 902 with a SERS-layer 903 on the surfaces of the walls of the slot 902. By index matching the cladding-layer, being the SERS-layer 903, in the slot 902 in combination with the chosen physical dimensions of the waveguide 901, optimum properties for the interaction between the light field 904, 905 (including the evanescent mode) and analytes in close contact with the SERS-layer 903 can be reached. The slot 902 will split the optical field 905 which combined with internal reflection in the waveguide 901 will even further enhance the interaction between the light field and the analytes interacting with the SERS-layer 903. The use of the waveguide 901 with a slot 902 will increase the emitted SERS-signal and thereby increase detectability of traces of a drug substance.

The SERS-layer could be Silica-gel including SERS-active particles, such as gold or silver.

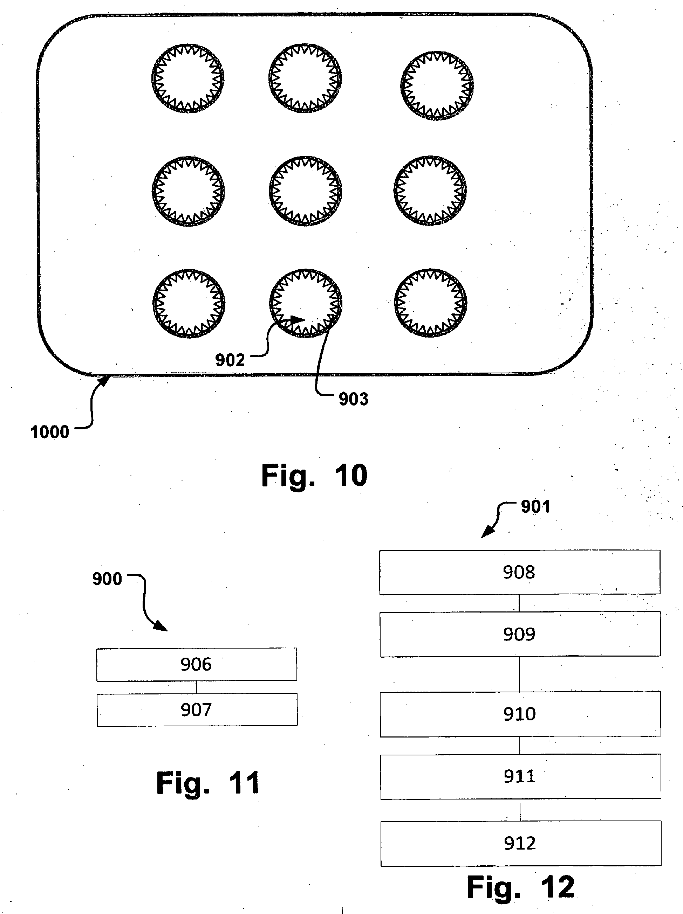

Fig. 11 is a flowchart illustrating an example for a method 91 of producing a slot for a SERS-sensor including the following steps:
906 Synthesis of amorphous silica with porogen.
907 Removal of porogen by heat treatment or chemical means.

Fig. 12 is a flowchart illustrating another example for a method 92 of producing a slot for a SERS-sensor including the following steps:.
908 Deposition of a first layer.
909 Deposition of a second layer.
910 Making holes by lithography in second layer.
911 Wet or dry-etching of first layer.
912 Wet or dry-etching of second layer.

Alternatively and/or additionally to the gels and slurries disclosed the silica-gel could be porous to further enhance the interaction between the drug compounds and the SERS-active surfaces.

Alternatively and/or additionally, in some embodiments a filter membrane, preferably an electrostatic non-woven filter membrane made of a blend of synthetic fibers such as acrylic fibers and polypropylene, could be used to collect the drug compounds from exhaled breath. The filter membrane could directly after being used be heated and the content in the filter membrane be evaporated onto a SERS-active surface. The filter is disposable and need to be changed after a certain amount of time used such as 10, 5 or 1.

The table below shows a test using a silica gel filter for sampling of methadone in exhaled breath from thee subjects. Showing that silica-gel with SERS-particles can be used.

| Experiment no | Methadone pg/min mean±SD | Range | n |
|---|---|---|---|
| 1 | 204±246 | 57-488 | 3 |

The detectable drug substance may be including in the non-exhaustive list comprising Amphetamines, ecstasy, Cannabis (THC and cannabinoids), Opiates heroin/morphine, 6-AM), Cocaine, Benzodiazepines, Propoxyphene, Methadone, Buprenorphine, Tramadol, LSD, Designer/Internet drugs, Kathinon, GHB, Meprobamat, Z-drugs, Tryptamines, Anabolic steroids, Alcohol/markers but are not limited to these. Other illicit drugs not included in the list could also be detectable due to similar interchanges with the human body as the above mentioned illicit drug substances.

Fig. 10 shows a schematic illustration of an embodiment of a plate 1000 of porous material, such as AIN, Al2N3 or Al203 with multiple channels, here holes 902. Active SERS particles such as Au or Ag nano-sized beads are attached to the walls of the holes 902 to obtain an active SERS surface 903. When breath is exhaled through the channels in the plate, over the SERS surface, illicit drug substances adhered to the walls and an emitted SERS signal can be detected. The SERS-signal occurs when these areas are illuminated by a light source e.g a diode laser.

In accordance with the present invention the disclosed SERS-probe is used in a drug interlock method for cars. The interlock method could work in different ways:
In one embodiment a mouthpiece is used into which the subject (in this case the driver) has to exhale. After exhaling into the mouthpiece the system may use any of the different SERS-devices disclosed within the context of this application or any other suitable detection system. After exhaling the system will determine if the driver can use the car or if the driver is not capable of handling the car, for example the driver is under the influence of a non-suitable substance such a drug or alcohol. The mouthpiece is preferably disposable and/or comprises bacterial killing substances such as silver or copper particles.

Alternatively and/or additionally, in some embodiments of the drug interlock the subject does not need to exhale into a mouthpiece. Around the coupé of the car may sensors be placed to detecting the presence and/or the amount of drug compounds at different locations around the coupé. In some embodiments the sensors are located close to the seat of the driver to detect if the driver is under the influence of a substance. In some embodiments the driver than has to exhale in the direction of the sensors.

The measurements could be enhanced by removing the background of the coupé to take into account if any of the passengers are under influence of a drug. The Background could be obtained using at least one senor located somewhere else in the coupé.

Additionally and or alternatively, in some embodiments the detection is performed by using the sensors to map the coupé to determining where a person under influence is seated. Alternatively and/or additionally, in some embodiments the mapping is further enhanced by using information from other sensors available in the car, such as sensors detecting if a seat is occupied, detection of eye movement etc. Other sensors that can be used are sensors to detecting the heartbeats and comparing them to the exhaling.

Alternatively and/or additionally the sensors could all have thereon detector and light source, preferably the detector and light source are shared by all sensors. This could be done by having optic fibers transmitting the emitted SERS signal to a central detector. When installing a sensor system the optical fibers could be laid in the infrastructure for cables already available in the car.

Alternatively and/or additionally, in some embodiments the sensors have to be replaced at some point. Either after the system has indicated the presence of a drug substance for a specific number of times or when the sensors have reached their "best before date". When changing the sensor only the waveguide with the SERS-layer is replaced.

Alternatively and/or additionally, in some embodiments klarite substrates are used for the detection of drug substances in a coupé of a car. If klarite substrates being used, than the substrate needs to be replaced.

In some embodiments, are the sensors automatically replaced by using a magazine comprising unused sensors i.e. waveguides with SERS-layer. The whole magazine may than be replaced when all have been used. An advantage using a non-mouthpiece based drug detection system is that the driver cannot ask someone else to exhale in his place, such as a passenger not under influence.

The present invention has been described above with reference to specific embodiments. However, other embodiments than the above described are equally possible within the scope of the invention. Different method steps than those described above, performing the method by hardware or software, may be provided within the scope of the invention. SERS-layer described as present on the outside of waveguides may likewise be present on an interior surface of a hollow waveguide, etc. The different features and steps of the invention may be combined in other combinations than those described. The scope of the invention is only limited by the appended patent claims.

## Claims

1. A method of interlocking a vehicle for preventing a subject under drug influence to operate said vehicle, **characterized in that** said method comprising:
providing at least one SERS-sensor in a vehicle interlock system,
exposing a SERS-active layer (903) of said SERS-sensor to exhaled breath,
analyzing if at least one analyte is present in exhaled breath of a subject based on measurement of said exhaled breath by means of said SERS-sensor,
and interlocking said vehicle if said analyte is detected in said exhaled breath.

2. Method of claim 1, wherein the vehicle is a motor vehicle comprising a coupé and at least a seat and the subject is a driver or passenger providing said exhaled breath.

3. Method of claim 1 or 2, wherein said analyte is a drug, and said analyzing comprises analyzing if said drug is present.

4. Method of claim 3, further comprising detecting if alcohol is present in said exhaled breath and said interlocking comprising interlocking said vehicle if alcohol and/or said drug is detected.

5. Method of any of claims 1 or 2, wherein sampling of said exhaled breath is made at multiple locations in said vehicle.

6. Method of claim 5, wherein said locations are located close to a seat of a driver of said vehicle for detecting if said driver is under the influence of said at least one analyte.

7. Method of claim 5, comprising determining where said subject under influence is seated in said vehicle based on mapping of analyzed analytes to said sampling locations.

8. Method of claim 7, comprising detecting if a driver and/or other passengers of said vehicle are under influence of said analyte, and interlocking said vehicle only if at least said driver is under influence of said analyte.

9. Method of claim 1, wherein emitted SERS signals are transmitted from multiple-SERS surface to a single detector.

10. Method of claim 1, comprising storing the number of times an analyte is detected by said SERS-sensor, and interlocking said vehicle when a predefined number is reached, or when said SERS-sensor has reached a predefined time of use, until at least a portion of said SERS-sensor is replaced, such as by changing a waveguide with a SERS-layer of said SERS-sensor, or a Klarite substrate thereof is replaced with an unused Klarite substrate.

11. A method of any of claim 1 to 10, further comprising:
- collecting an exhaled breath sample from said subject in said vehicle;
- making contact between said exhaled breath sample and a SERS-active layer of said SERS-sensor;
- recording at least one SERS-enhanced Raman spectrum obtained from said SERS-sensor.

12. Method of claim 11, comprising:
- recording at least one background spectrum using the SERS-sensor;
- collecting said exhaled breath sample in a chamber;
- recording a spectrum of the exhaled breath sample using said SERS-sensor;
- analyzing said spectrum; and
- based on said analysis giving an indication if said analyte is present in said exhaled breath via a user interface.

13. Method of claim 1, said SERS-sensor comprising a light source, a light detector, at least one optical filter, and at least one planar multimode waveguide having a slot, and a collecting surface having at least one Surface Enhanced Raman Spectroscopy (SERS)-active cladding layer being refractive index matched with said planar waveguide, said collecting surface is arranged as an outer surface in said slot for contact with said exhaled breath, for bringing said exhaled breath in close contact said SERS-active layer, and
said at least one planar waveguide is coupled to said light source and said detector, such that an optic field from said light source and an evanescent mode are interacting with said analyte in close contact with said SERS-layer;
and enhancing, by total internal reflection in said waveguide and said slot, both said optic field and said evanescent mode interaction for detecting a SERS-emitted signal from said analytes; and detecting a Raman shift spectrum by a presence of said analyte, and/or determining the quantitative amount thereof, from said exhaled breath.

14. Method of claim 1, comprising build up of a multivariate data model for recognition of a spectral pattern related to target molecules of said analyte, preferably in the wavenumber range of 600 - 3000 cm⁻¹, such as 600 - 1800 cm⁻¹ or around 2300 or around 3000 cm⁻¹.

## Patentansprüche

1. Verfahren zur Verriegelung eines Fahrzeugs zum Zwecke der Vermeidung, dass eine unter Drogeneinfluss stehende Testperson das Fahrzeug bedient, **dadurch gekennzeichnet, dass** das Verfahren umfasst;
Bereitstellung von mindestens einem SERS-Sensor in einem Fahrzeugsperrsystem,
Einwirkung der Ausatemluft auf mindestens eine SERS-aktive Schicht (903) eines SERS-Sensors,
Analyse, ob mindestens ein Analyt in der Ausatemluft der Testperson vorhanden ist, was basierend auf einer mittels eines SERS-Sensors durchgeführten Messung der Ausatemluft erfolgt,
und Sperrung des Fahrzeugs, sofern der Analyt in der Ausatemluft festgestellt wird.

2. Verfahren gemäß Anspruch 1, wobei es sich bei dem Fahrzeug um ein Motorfahrzeug handelt, welches ein Coupé und mindestens einen Sitz umfasst, und wobei es sich bei der Testperson um einen Fahrer oder einen Passagier handelt, von welchem die Ausatemluft stammt.

3. Verfahren gemäß Anspruch 1 oder 2, wobei es sich bei dem Analyt um eine Droge handelt, und die Analyse eine Analyse zur Feststellung des Vorhandenseins der Droge umfasst.

4. Verfahren gemäß Anspruch 3, ferner die Feststellung umfassend, ob Alkohol in der Ausatemluft vorhanden ist, und wobei die Sperrung eine Sperrung des Fahrzeugs umfasst, sofern Alkohol und/oder die Droge festgestellt wird.

5. Verfahren gemäß einem beliebigen der Ansprüche 1 oder 2, wobei die Probeentnahme der Ausatemluft an mehreren Stellen in dem Fahrzeug durchgeführt wird.

6. Verfahren gemäß Anspruch 5, wobei sich diese Stellen zum Zwecke der Erkennung, ob der Fahrer unter dem Einfluss von mindestens einem Analyt steht, in der Nähe des Sitzes eines Fahrers des Fahrzeugs befinden.

7. Verfahren gemäß Anspruch 5, die Feststellung umfassend, wo die Testperson, welche unter dem Einfluss steht, in dem Fahrzeug sitzt, was basierend auf einer Zuordnung der analysierten Analyte zu den Probeentnahme-Stellen geschieht.

8. Verfahren gemäß Anspruch 7, die Feststellung umfassend, ob ein Fahrer und/oder andere Passagiere des Fahrzeugs unter dem Einfluss des Analyts stehen, und die Sperrung des Fahrzeugs nur dann umfassend, wenn mindestens der Fahrer unter dem Einfluss des Analyts steht.

9. Verfahren gemäß Anspruch 1, wobei emittierte SERS-Signale von mehreren SERS-Oberfläche[n] an einen einzigen Detektor übertragen werden.

10. Verfahren gemäß Anspruch 1, die Speicherung der Anzahl von Malen umfassend, die ein Analyt vom SERS-Sensor festgestellt wird, und Verriegelung des Fahrzeugs, wenn eine zuvor festgelegte Anzahl erreicht worden ist, oder wenn der SERS-Sensor eine zuvor definierte Anzahl von Benutzungen erreicht hat, bis mindestens ein Abschnitt des SERS-Sensors ersetzt worden ist, wie beispielsweise durch Auswechseln eines Wellenleiters mit einer SERS-Schicht des SERS-Sensors, oder indem ein Klarit-Substrat von diesem durch ein ungebrauchtes Klarit-Substrat ersetzt wird.

11. Verfahren gemäß einem beliebigen von Ansprüche 1 bis 10, ferner umfassend:
- Entnahme einer Ausatemluft-Probe von der Testperson in dem Fahrzeug;
- Herstellung eines Kontakts zwischen der Ausatemluft-Probe und der SERS-aktiven Schicht des SERS-Sensors;
- Aufzeichnung von mindestens einem SERS-verstärkten Raman-Spektrum, welches von dem SERS-Sensor erfasst wurde.

12. Verfahren gemäß Anspruch 11, umfassend:
- Aufzeichnung von mindestens einem Hintergrundspektrum unter Verwendung eines SERS-Sensors;
- Sammeln der Ausatemluft-Probe in einer Kammer;
- Aufzeichnung eines Spektrums der Ausatemluft-Probe unter Verwendung des SERS-Sensors;
- Analyse des Spektrums; und
- Ausgabe eines auf der Analyse beruhenden Hinweises über eine Benutzeroberfläche, ob der Analyt in der Ausatemluft vorhanden ist.

13. Verfahren gemäß Anspruch 1, wobei der SERS-Sensor eine Lichtquelle, einen Lichtdetektor, mindestens einen optischen Filter und mindestens einen planaren Multimode-Wellenleiter mit einem Schlitz sowie eine Sammeloberfläche mit mindestens einer oberflächenverstärkten ramanspektroskopie(SERS)-aktiven Mantelschicht aufweist, die in Bezug auf ihren Brechungsindex auf den planaren Wellenleiter angepasst ist, wobei die Sammeloberfläche als eine Außenfläche in dem Schlitz angeordnet ist, damit sie mit der Ausatemluft in Kontakt kommt, so dass die Ausatemluft in engen Kontakt mit der SERS-aktiven Schicht gebracht wird, und
wobei der mindestens eine planare Wellenleiter mit der Lichtquelle und dem Detektor gekoppelt ist, so dass ein optisches Feld von der Lichtquelle und ein evaneszenter Modus mit dem Analyt, welcher in engem Kontakt mit der SERS-Schicht steht, zusammenwirken;
und Verbesserung sowohl des Zusammenwirkens des optischen Feldes mit dem evaneszenten Modus zur Erkennung des SERS-emittierten Signals von den Analyten; als auch der Erkennung eines Raman-Verschiebungs-Spektrums durch das Vorhandensein des Analyts, und/oder zur Feststellung einer quantitativen Menge von diesem anhand der Ausatemluf, mittels Totalreflexion in dem Wellenleiter und dem Schlitz.

14. Verfahren gemäß Anspruch 1, den Aufbau eines multivariaten Datenmodells zur Erkennung eines spektralen Musters umfassend, welches mit den Zielmolekülen des Analyts im Zusammenhang steht, vorzugsweise in dem Wellenanzahl-Bereich von 600 - 3000 cm⁻¹, wie beispielsweise 600 -1800 cm⁻¹ oder um 2300 oder um 3000 cm⁻¹ herum.

## Revendications

1. Procédé de verrouillage d'un véhicule destiné à empêcher un sujet sous influence d'une drogue d'utiliser ledit véhicule, **caractérisé en ce que** ledit procédé comprend les étapes consistant à :
fournir au moins un capteur SERS dans un système de verrouillage de véhicule,
exposer une couche active SERS (903) dudit capteur SERS à un souffle exhalé,
analyser si au moins un analyte est présent dans le souffle exhalé d'un sujet à partir d'une mesure dudit souffle exhalé au moyen dudit capteur SERS
et verrouiller ledit véhicule si ledit analyte est détecté dans ledit souffle exhalé.

2. Procédé selon la revendication 1, dans lequel le véhicule est un véhicule à moteur comprenant un coupé et au moins un siège et le sujet est un conducteur ou un passager fournissant ledit souffle exhalé.

3. Procédé selon la revendication 1 ou 2, dans lequel ledit analyte est une drogue, et ladite analyse comprend le fait d'analyser si ladite drogue et présente.

4. Procédé selon la revendication 3, comprenant en outre l'étape consistant à détecter si de l'alcool est présent dans ledit souffle exhalé et ledit verrouillage comprenant le verrouillage dudit véhicule si l'alcool et/ou ladite drogue est détecté.

5. Procédé selon l'une quelconque des revendications 1 ou 2, dans lequel l'échantillonnage dudit souffle exhalé est réalisé à plusieurs positions dans ledit véhicule.

6. Procédé selon la revendication 5, dans lequel lesdites positions sont situées à proximité d'un siège d'un conducteur dudit véhiculent pour détecter si ledit conducteur est sous l'influence dudit au moins un analyte.

7. Procédé selon la revendication 5, comprenant la détermination de l'endroit où ledit sujet sous influence est assis dans ledit véhicule à partir du mappage d'analytes analysés sur lesdites positions d'échantillonnage.

8. Procédé selon la revendication 7, comprenant une détection du fait qu'un conducteur et/ou d'autres passagers dudit véhicule sont sous l'influence dudit analyte, et le verrouillage dudit véhicule seulement si ledit conducteur est sous l'influence dudit analyte.

9. Procédé selon la revendication 1, dans lequel les signaux SERS émis sont transmis à partir de plusieurs surfaces SERS vers un seul détecteur.

10. Procédé selon la revendication 1, comprenant la mémorisation du nombre de fois qu'un analyte est détecté par ledit capteur SERS, et le verrouillage dudit véhicule lorsqu'un nombre prédéterminé est atteint, ou lorsque ledit capteur SERS a atteint un temps d'utilisation prédéfini, jusqu'à ce qu'au moins une partie dudit capteur SERS soit remplacée, par exemple en changeant un guide d'ondes avec une couche SERS dudit capteur SERS, ou un substrat de Klarite de celui-ci est remplacé par un substrat de Klarite non utilisé.

11. Procédé selon l'une quelconque des revendications 1 à 10, comprenant en outre les étapes consistant à :
- collecter un échantillon de souffle exhalé à partir dudit sujet dans ledit véhicule,
- réaliser un contact entre ledit échantillon de souffle exhalé et une couche active SERS dudit capteur SERS,
- enregistrer au moins un spectre de Raman exalté SERS obtenu à partir dudit capteur SERS.

12. Procédé selon la revendication 11, comprenant les étapes consistant à :
- enregistrer au moins un spectre de bruit de fond en utilisant le capteur SERS,
- collecter ledit échantillon de souffle exhalé dans une chambre,
- enregistrer un spectre de l'échantillon de souffle exhalé en utilisant ledit capteur SERS,
- analyser ledit spectre, et
- sur la base de ladite analyse donner une indication du fait que ledit analyte est présent dans ledit souffle exhalé par le biais d'une interface utilisateur.

13. Procédé selon la revendication 1, ledit capteur SERS comprenant une source de lumière, un détecteur de lumière, au moins un filtre optique, et au moins un guide d'ondes multimode plan ayant une fente, et une surface de collecte ayant au moins une couche de placage active de spectroscopie de Raman exaltée de surface (SERS) dont l'indice de réfraction correspond à celui dudit guide d'ondes plan, ladite surface de collecte est prévue sous la forme d'une surface extérieure dans ladite fente pour un contact avec ledit souffle exhalé, pour amener ledit souffle exhalé en contact direct avec ladite couche active SERS, et
ledit au moins un guide d'ondes plan est couplé à ladite source de lumière et audit détecteur de lumière, de sorte qu'un champ optique provenant de ladite source de lumière et un mode évanescent interagissent avec ledit analyte en contact direct avec ladite couche SERS,
et l'amélioration, par une réflexion interne totale dans ledit guide d'ondes et ladite fente, à la fois dudit champ optique et de ladite interaction de mode évanescent pour une détection d'un signal émis SERS à partir desdits analytes, et la détection d'un spectre de décalage de Raman par une présence dudit analyte, et/ou la détermination de sa valeur quantitative, à partir dudit souffle exhalé.

14. Procédé selon la revendication 1, comprenant la construction d'un modèle de données à plusieurs variables pour une reconnaissance d'un motif spectral lié à des molécules cibles dudit analyte, de préférence dans la plage de longueurs d'ondes de 600 à 3000 cm⁻¹, par exemple de 600 à 1800 cm⁻¹ ou autour de 2300 cm⁻¹ ou autour de 3000 cm⁻¹.
